# EUROPEAN PATENT APPLICATION

(11) **EP 4 174 869 A1**
(43) Date of publication of application: **03.05.2023**
(21) Application number: 22162114.7
(22) Date of filing: 15.03.2022
(51) Int. Cl.: G16H 40/60, G09B 23/00

(54) **CASE-BASED MIXED REALITY PREPARATION AND GUIDANCE FOR MEDICAL PROCEDURES**

(30) Priority: 26.10.2021 US 202163271876 P
(71) Applicant: Koninklijke Philips N.V., 5656 AG Eindhoven (NL)
(72) Inventor: SHRUBSOLE, Paul Anthony, Eindhoven (NL); DU, Jia, Eindhoven (NL); KOKS, Yvonne, Eindhoven (NL)
(74) Representative: Philips Intellectual Property & Standards

(57) **Abstract**

A non-transitory computer readable medium (26) stores instructions executable by at least one electronic processor (20) to perform a mixed reality medical procedure simulation method (100). The method includes: receiving, via one or more user inputs from a user with at least one user input device (22), a medical procedure the user wants to simulate; identifying, in a simulations database (30), a mixed reality medical procedure educational simulation (32) similar to the medical procedure the user wants to simulate; performing a risk analysis on the medical procedure the user wants to simulate to generate a risk model (34) for the medical procedure the user wants to simulate; generating a user-specific mixed reality medical procedure simulation (12) by augmenting the selected mixed reality medical procedure simulation with the risk model; and presenting the user-specific mixed reality medical procedure simulation to the user.

## Description

### FIELD OF THE INVENTION

The following relates generally to the medical training arts, educational content arts, augmented reality (AR) arts, AR medical training content generation and delivery arts, and related arts.

### BACKGROUND OF THE INVENTION

Training for medical procedures can include using simulation methods to teach and assess technical skills before a medical resident performs procedures on patients. Such simulation methods can include mixed reality ("MR", e.g., augmented reality (AR), virtual reality (VR), or a combination thereof). Existing mixed reality simulations of medical procedures focus on trying to reproduce the anatomical aspects of the procedure, but do not focus on the case-specific aspects or on guidance that relates to these specific cases and how to deal with difficult situations.

Surgeons, interventional radiologists, or other medical professionals that require training on medical procedures are often presented with specific patient cases that are somewhat unfamiliar to them. This unfamiliarity presents a degree of risk to the patient during the intervention and could result in complications (e.g., multi-morbidity combinations, immune-deficiencies, organ failure, and so forth). Currently there are limited tools to sufficiently prepare for these cases and simulate how to manage the risk when something can go wrong.

Due to events, such as the COVID-19 pandemic, more and more activities that used to be done in a direct, face-to-face format are transferring to remote and virtual simulated context. These can be done in a simple audio/video call between two persons, or a conference call session involving more people. This can also take place in a mixed reality environment. Depending on the design, the mixed reality environment may be a fully virtual environment or an augmented reality environment. In an augmented reality environment, a physical object such as a mannequin serves as the "subject" of an operation, and the user wears augmented reality eyewear with transparent displays enabling superimposing virtual content onto the real physical world (e.g., superimposing a surgical site onto the mannequin for example). In a fully virtual environment, the user wears a virtual reality headset or the like which presents a completely virtual surgical setting (or other medical setting) divorced from the real world. In either case, the user may wear gloves with sensors (e.g., finger bend sensors, accelerometers, or so forth), manipulate surgical instruments or apparatus with sensors, or other user input hardware by which actions such as hand and instrument motions are translated into the mixed reality (e.g., AR or VR) environment.

The following discloses certain improvements to overcome these problems and others.

### SUMMARY OF THE INVENTION

In one aspect, a non-transitory computer readable medium stores instructions executable by at least one electronic processor to perform a mixed reality medical procedure simulation method. The method includes: receiving, via one or more user inputs from a user with at least one user input device, a medical procedure the user wants to simulate; identifying, in a simulations database, a mixed reality medical procedure educational simulation similar to the medical procedure the user wants to simulate; performing a risk analysis on the medical procedure the user wants to simulate to generate a risk model for the medical procedure the user wants to simulate; generating a user-specific mixed reality medical procedure simulation by augmenting the selected mixed reality medical procedure simulation with the risk model; and presenting the user-specific mixed reality medical procedure simulation to the user.

In another aspect, a non-transitory computer readable medium stores instructions executable by at least one electronic processor to perform an augmented reality (AR) medical procedure education method. The method includes: receiving, via one or more user inputs from a user with at least one user input device, a medical procedure the user wants to learn; identifying, in a simulations database, a mixed reality medical procedure educational simulation similar to the medical procedure the user wants to learn; performing a risk analysis on the medical procedure the user wants to learn to generate a risk model for the medical procedure the user wants to learn by quantifying one or more risk factors based on an expertise level of the user; generating a user-specific mixed reality medical procedure educational simulation by augmenting the selected mixed reality medical procedure educational simulation with the risk model; and presenting the user-specific mixed reality medical procedure educational simulation to the user.

In another aspect, a mixed reality medical procedure education method includes: receiving, via one or more user inputs from a user with at least one user input device, a medical procedure the user wants to learn; identifying, in a simulations database, a mixed reality medical procedure educational simulation similar to the medical procedure the user wants to learn; performing a risk analysis on the medical procedure the user wants to learn to generate a risk model for the medical procedure the user wants to learn by analyzing log data of a medical device to be used in the medical procedure the user wants to learn and historical patient data of historical patients who have undergone the medical procedure the user wants to learn and quantifying the one or more risk factors based on the analyzing of the log data and the historical patient data; generating a user-specific mixed reality medical procedure educational simulation by augmenting the selected mixed reality medical procedure educational simulation with the risk model; and presenting the user-specific mixed reality medical procedure educational simulation to the user.

One advantage resides in providing mixed reality educational materials for medical professionals to learn about medical procedures, which are tailored to specific patients or clinical situations.

Another advantage resides in providing mixed reality educational materials for medical professionals to learn about medical procedures based on knowledge, expertise, and/or experience of the medical professionals.

Another advantage resides in providing mixed reality educational materials for medical professionals in real-time during a medical procedure.

Another advantage resides in providing mixed reality educational materials for medical professionals during a medical procedure on a headset worn by the medical professional.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically illustrates an illustrative system for providing a user-specific mixed reality medical procedure educational simulation in accordance with the present disclosure.
Fig. 2 shows exemplary flow chart operations of the system of Fig. 1.

### DETAILED DESCRIPTION

The following relates to approaches for leveraging augmented reality (AR) or virtual reality (VR), collectively referred to herein as mixed reality (MR), for training surgeons or other medical professionals to perform medical procedures.

The disclosed system employs a recording module to record experts performing various medical procedures, with various types of risks. The recordings may be of procedures performed on actual patients, and/or simulated procedures performed on anatomical dummies. Notably, the recordings include sensor information from the various computerized instruments used in performing the recorded procedure as well as case data from a hospital information system. Recordings may also optionally include a visual component provided, for example, using cameras mounted on eyeglasses or goggles worn by the expert while performing the procedure. In some embodiments, the cameras are three-dimensional (3D) range cameras that capture 3D content for generating a 3D mixed reality environment. Recordings may also be annotated with patient details of the actual patient (for procedures performed on actual patients) or for the simulated patient (for procedures performed on anatomical dummies). The collection of such recordings of medical procedures, over time, generates a database of recorded mixed reality procedures for a wide range of medical procedures performed on patients or anatomical dummies having or simulating specific risk factors.

In this regard, the database is unlikely to include many (or any) specific examples in which a problem actually arose due to a risk factor, especially where the risk factor involved is of low probability. It may not be cost-effective to simulate such unusual cases using an anatomical dummy, and in the case of actual patients the likelihood of the database containing an unusual case is also low. Hence, the database is likely to include mostly "normal" examples, in which the procedure was done routinely. Similarly, the database may be unlikely to contain examples of mistakes of the type more likely to be made by a less experienced medical professional. Typically, "good" examples of medical procedures performed by experts in the procedures are recorded, so as to provide model procedures for the student to learn to emulate.

However, it is recognized herein that mixed reality educational materials will often be consumed by surgeons or other medical professionals who need to prepare for a challenging operation or other challenging medical procedure. The challenge may arise due to an unusual circumstance of a particular case, such as a patient with an unusual risk factor. The unusual circumstance may produce an elevated likelihood of a certain complication or complications that could occur during the medical procedure in this particular case, which are unlikely to be captured in the model mixed reality educational procedure example. In other cases, the challenge may arise due to inexperience of the surgeon or other medical professional, who may be at elevated risk of making certain mistakes that a more experienced medical professional would be unlikely to make. In other cases, the challenge may arise due to inexperience of the surgeon or other medical professional with a particular medical instrument to be used in the procedure, especially if that particular medical instrument is not commonly used in the subject medical domain and/or its handling is unusual. Again, there may be no model mixed reality medical procedure examples in the database that cover that particular medical instrument.

To provide mixed reality educational simulations that more effectively address these types of situations, it is disclosed herein to perform a risk analysis on the medical procedure the user wants to learn to generate a risk model for the medical procedure the user wants to learn. The risk analysis may for example be based on one or more risk factors of a specific patient on whom the user wants to perform the medical procedure the user wants to learn, risk factors determined based on an expertise level of the user, and/or so forth. A user-specific mixed reality medical procedure educational simulation is then generated by augmenting the closest mixed reality medical procedure educational simulation in the database with the risk model. The user-specific mixed reality medical procedure educational simulation is then presented to the user.

In some embodiments disclosed herein, a case selector is provided, which a student uses to input details of a case the student wants to practice or be instructed. The student (or more generally, "user") may be a medical student, or a working professional (e.g. a surgeon) who is preparing for an upcoming specific procedure having some aspect(s) the surgeon is unfamiliar with. The case selector preferably allows the student to enter detailed information about the procedure, the specific patient's risk factors, and the student's level expertise. The latter expertise input may be obtained indirectly based on the student's accreditation (e.g., medical student or practicing surgeon), and/or may be obtained by the student affirmatively identifying aspect(s) the student wants to focus on. Based on this information the case selector chooses the most relevant recorded mixed reality procedure from the database.

Prior to presenting the educational content, the risk assessment is performed. The risk assessment is preferably based on actual machine log data and actual patients, so that it accurately reflects statistics of actual patient risks for the specific medical instruments being used in actual situations. Risk factors can thus be statistically quantified. Additionally, the risk factors can be adjusted based on the student's experience. For example, if the student is a practicing surgeon who has performed this type of procedure many times previously, but not on patients having a specific risk presenting in the upcoming patient, then the risk factor for that specific risk can be upweighted, while risk factors for risks presenting in many or all patients can be down-weighted due to the surgeon's previous experience. On the other hand, if the user has limited experience in the medical procedure, then more commonplace (e.g., not patient-specific) risks may be upweighted over less common risks. As another example, if the user will be performing the medical procedure using a make/model of medical instrument that is different from the make/model of medical instrument used in the most relevant recorded mixed reality procedure selected from the database, then risks associated with the make/model of medical instrument that will be used in the procedure can be considered as a risk factor in the analysis.

Next, the patient- and student-specific simulation is generated by augmenting the mixed reality medical procedure educational simulation selected from the database with the risk model, and the resulting user-specific mixed reality medical procedure educational simulation is presented to the user. This generally will include playback of the selected most relevant recorded mixed reality procedure, augmented by the patient- and student-specific risk factors. Based on the risk factors, for example, a contrast agent flow rate and/or time may be adjusted in the specific simulation being created. The specific simulation may also include time adjustments (speeding up or slowing down) for certain phases of the procedure to reflect the estimated time that should be taken to perform those phases in the specific patient given the skill level of the student.

Finally, a playback module plays back the most relevant recorded mixed reality procedure, with the risk-factor-based augmentation. The student typically wears a VR/AR headset or the like, via which the playback is presented. Various patient- and student-specific adjustments may be made in this playback.

In some embodiments, the playback is entirely passive, i.e. the student watches the mixed reality playback but does not interact with it. In this case, the mixed reality playback may be 100% virtual reality (VR), and may include textual or graphical annotations indicating points in the procedure that are impacted by risks with large risk factors. In the previous example, when contrast agent is administered a text or graphic may appear emphasizing the criticality of the contrast agent flow rate and/or time.

In some embodiments, the mixed reality playback is more interactive, employing augmented reality (AR) playback superimposed on a real training setting. For example, the student may use a catheter or other medical instrument to actually perform the procedure on an anatomical dummy. In this case, the mixed reality playback may include superimposing augmented reality video of playback of the recorded procedure on the view of the anatomical dummy seen by the student through the AR headset. Here, the options for augmentation are more diverse. For example, in addition to the aforementioned superposition of text or graphics noting procedure points impacted by high risk factors, the "feed forward" augmentation can additionally include text or graphics warning the student when the actually performed procedure deviates from the recorded playback. For example, if the student is using the incorrect contrast agent delivery settings, then the mixed reality content text or graphics can point this out, along with an explanation of why the student's settings are incorrect, potential consequences of the error, as well as displaying the correct settings.

As another example, if the student uses the incorrect settings on the contrast agent pump this could be highlighted by superimposing the correct settings as mixed reality content. If the student is performing a phase of the procedure too quickly then the correct-speed simulation can be superimposed as mixed reality content to encourage the student to slow down; or vice versa if the student is performing a phase too quickly. Such a superposition can, for example, appear as a "ghost", i.e. semi-transparent mixed reality content which the student can see but also through which the student can see the actual physical anatomical dummy.

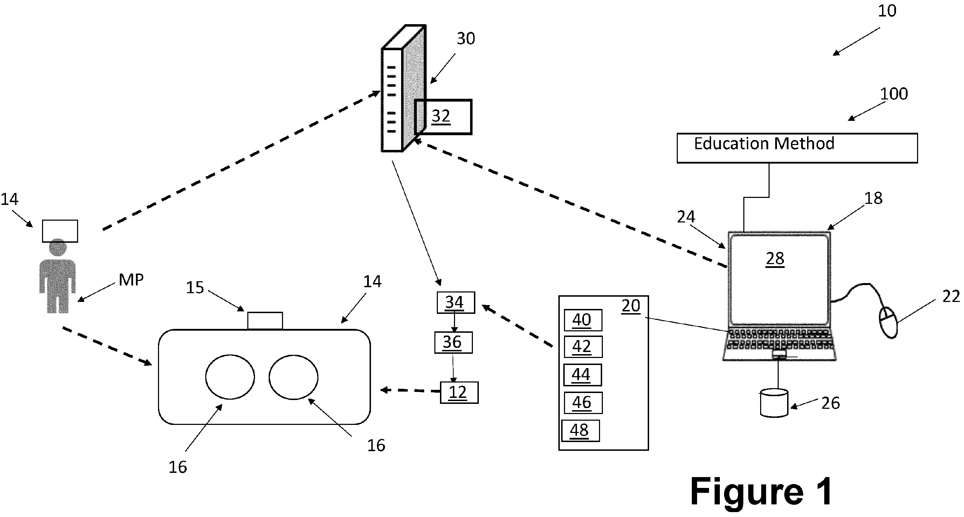

With reference to Fig. 1, an illustrative apparatus or system 10 for generating and presenting a user-specific mixed reality medical procedure educational simulation 12 to a medical professional (MP) (e.g., a physician, a nurse, a technician, and so forth) to perform a medical procedure. In some embodiments, the user-specific mixed reality medical procedure educational simulation 12 can be output on a headset 14 (e.g., a VR or AR heads-up display (AR-HUD) or AR glasses worn by the MP (e.g., during a medical procedure, or before a medical procedure). The headset 14 can have a camera 15 affixed thereto to project a rendering of the user-specific mixed reality medical procedure educational simulation 12 into a viewpoint of the MP. The headset 14 can include a display device 16 to displaying the user-specific mixed reality medical procedure educational simulation 12. Optionally, further input may be provided by gloves worn by the medical professional MP and/or medical instruments or the like (not shown) that include sensors for measuring finger positions, hand position/orientation, instrument orientation, or other relevant information during a medical procedure.

Fig. 1 also shows an electronic processing device **18**, such as a workstation computer, a smartphone, a tablet, or so forth, configured to generate and present the user-specific mixed reality medical procedure educational simulation **12.** Additionally or alternatively, the electronic processing device **18** can be embodied as a server computer or a plurality of server computers, e.g. interconnected to form a server cluster, cloud computing resource, or so forth. In other embodiments, the electronic processing device **18** can be integrated into the headset(s) **14.** In further embodiments, the electronic processing device **18** can be connected to the headset(s) **14** via a wireless communication network (i.e., the Internet).

The electronic processing device **18** optionally includes typical components, such as an electronic processor **20** (e.g., a microprocessor), at least one user input device (e.g., a mouse, a keyboard, a trackball, and/or the like) **22**, and at least one display device **24** (e.g. an LCD display, an OLED display, a touch-sensitive display, plasma display, cathode ray tube display, and/or so forth). In some embodiments, the display device **24** can be a separate component from the electronic processing device **18.** The display device **24** may also comprise two or more display devices. However, in some embodiments these conventional user interfacing components may be omitted since user interfacing is provided by the previously described headset(s) **14** and sensors in gloves, medical instruments, and/or so forth.

The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the electronic processing device **18**, various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the display device **24.**

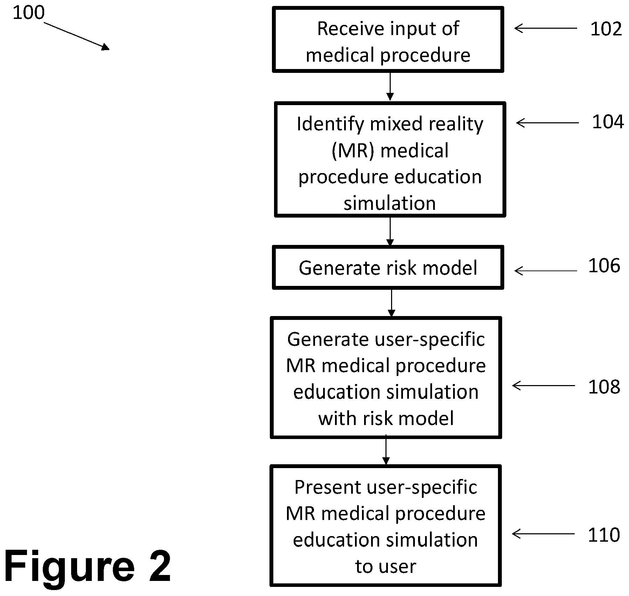

The electronic processing device **18** (and/or the headset **14**) is connected with a database **30** via a wireless communication network (i.e., the Internet). The database **30** comprises a simulations database **30** storing a plurality of mixed reality medical procedure educational simulation **32**, which can be used as educational content to be accessed and reviewed by the **MP.** To generate the mixed reality medical procedure educational simulations **32**, **MPs** can use a simulation recorder module **40** (implemented in the headset **14** or the electronic processing device **18**) to record procedures of specific cases for educational purposes (for privacy reasons, the patient's features are preferably automatically anonymized, e.g. modified to look generic according to the BMI, height etc.). The case-based simulation recorder module **40** can capture aspects of the medical procedure (e.g., spatial features, digital features, temporal features, and so forth) performed by the **MP**, which is then encoded for a later playback. The case-based simulation recorder module **40** aggregates information, such as patient data, machine log data, positional data of the **MP**, a view of the **MP** during the procedure, and so forth, and converts this data to a mixed reality format to generate the mixed reality medical procedure educational simulations **32.** Furthermore, as disclosed herein, the non-transitory storage media **26** performs a mixed reality medical procedure education (or simulation) method or process **100.** With reference to Fig. 2, and with continuing reference to Fig. 1, the electronic processing device **18** is configured as described above to perform the mixed reality medical procedure education method **100.** The non-transitory storage medium **26** stores instructions which are readable and executable by the electronic processing device **18** to perform disclosed operations including performing the method or process **100.** In some examples, the method **100** may be performed at least in part by cloud processing.

At an operation **102**, one or more user inputs from a user (i.e., the **MP**) about a medical procedure the **MP** wants to learn (or simulate) are received by the electronic processing device **18** (via the at least one user input device **22**) or the headset **14** (via a finger-tap or swipe on the display device **16**). At an operation **104**, a mixed reality medical procedure educational simulation **32** similar to the medical procedure the user wants to learn is identified in the simulations database **30.** As used herein, a "similar" mixed reality medical procedure educational simulation **32** refers to a mixed reality medical procedure educational simulation most similar to the medical procedure about which the **MP** wants to learn.

To perform the user input operation **102** and the identification operation **104**, a case selector module **42** is configured to provide the GUI **28** on the display device **24** of the electronic processing device **18** (or on the display device **16** of the headset **14**), to allow the **MP** to input details about the medical procedure. The user inputs may for example identify information such as the type of medical procedure to be performed, characteristics of the patient scheduled to undergo that procedure (especially characteristics that may introduced patient-specific risks), other relevant information such make/model of medical instrument(s) to be used in the procedure, information about the experience level of the user in performing the medical procedure, and/or so forth. The information may be entered, for example, in a structured data entry form with text-based and/or graphical structured, semi-structured, and/or free-form text entry fields. In some cases, additional information may be retrieved automatically from a patient record or other electronic database. For example, in response to entry of a patient identification (PID), that patient's electronic health record may be searched to retrieve relevant patient-specific risk information. The case selector module **42** can then detect that the current case is new by reviewing a history of previous cases (i.e., the mixed reality medical procedure educational simulations **32** stored in the simulations database **30**) against scheduled procedures for a medical facility. From a particular case, the most similar mixed reality medical procedure educational simulation **32** to the current case is identified. Various similarity metrics may be employed. For example, similarity factors may include similar or identical medical procedure type, similar or identical patient characteristics, similar or identical make/model of medical instrumentation, and so forth. In general, it typically will not be possible to identify an exact match in which every such similarity factor is identical in the selected similar mixed reality medical procedure educational simulation. If no sufficiently similar case is found, the **MPs** are automatically informed of such.

At an operation **106**, a risk analysis is performed on the medical procedure the user wants to learn to generate a risk model **34** for the medical procedure the user wants to learn. In some embodiments, the risk analysis is based on at least one of a risk factor **36** of a specific patient on whom the user wants to perform the medical procedure the user wants to learn. In other embodiments, the risk analysis includes analyzing log data of a medical device (not shown) to be used in the medical procedure the user wants to learn and historical patient data of historical patients who have undergone the medical procedure the user wants to learn. The risk factor(s) 36 are then quantified based on the analyzing of the log data and the historical patient data. For example, the risk factor(s) 36 are quantified based on an expertise level of the user.

To perform the risk analysis operation **106**, a risk assessment module **44** is configured to combine patient history data related to the selected mixed reality medical procedure educational simulation **32**, a number of similar cases handled by the hospital/physician and regional patient cohort/population data, and recordings of training sessions and courses that the **MP** has attended to create the risk model **34** associated with a specific planned intervention. The risk assessment module **44** may also receive specific risk information taken from the recordings of example procedures (e.g., through annotations from the **MP** who performed the procedure). The risk model **34** is then mapped to key events in the procedure timeline to form a hierarchical, time-based model of key events and possible outcomes with their associated probabilities.

At an operation **108**, the user-specific mixed reality medical procedure educational simulation **12** is generated by augmenting the selected mixed reality medical procedure educational simulation **32** with the risk model **34.** The augmenting can also include augmenting the selected mixed reality medical procedure educational simulation **32** with the quantified risk factor(s) **36.** For example, mixed reality content indicative of the risk factor(s) **36** at time points in the selected mixed reality medical procedure educational simulation **32** at which the quantified risk factor(s) **36** occur can be added to the selected mixed reality medical procedure educational simulation **32.**

To perform the generation operation **108**, a simulation creation module **46** is configured to generate the user-specific mixed reality medical procedure educational simulation **12** from the risk model **34** and the expertise of the **MP.**

At an operation **110**, the user-specific mixed reality medical procedure educational simulation **12** is presented to the **MP** (e.g., on the display device **16** of the headset **14**, on the display device **24** of the electronic processing device **18**, and so forth). In some embodiments, when the user-specific mixed reality medical procedure educational simulation **12** is presented (i.e., played back) to the **MP**, a user input (via the at least one user input device **22** or a finger-tap or swipe on the display device **16**) of the **MP** interacting with the user-specific mixed reality medical procedure educational simulation **12** is received. A speed of playback of the user-specific mixed reality medical procedure educational simulation **12** can be adjusted (e.g., sped up, slowed down, paused, and so forth) based on the user input. In other embodiments, during playback, a user input indicative of the user interacting with the user-specific mixed reality medical procedure educational simulation **12** can be received, where it is determined whether the received user input corresponds to a correct step in the medical procedure. If the received user input is determined to not correspond to a correct step, mixed reality content can be provided that the user input was not correct. For example, a corrective action, such as adjusting a speed of feedforward information on how to follow a correct action, can be provided.

To perform the presentation operation **110**, a simulation feedforward playback module **48** is configured to playback the user-specific mixed reality medical procedure educational simulation **12** so that important events in the medical procedure can be overlaid with mixed reality content for the **MP.** The events can be selected by, for example, determining an area of interest while the **MP** is performing the task, repeat or slow down playback of the user-specific mixed reality medical procedure educational simulation **12** when the **MP** makes a mistake during the playback, match the playback to the actual medical procedure context, and so forth.

### EXAMPLE

The following describes an example of the method 100 for a coronary angioplasty and stent insertion medical procedure using an IGT system. Some risk factors involved in angioplasty procedures can include bleeding or bruising under the skin where the catheter was inserted, damage to the artery where the sheath was inserted, an allergic reaction to the contrast agent used during the procedure, damage to an artery in the heart, excessive bleeding requiring a blood transfusion, heart attack, stroke, or death, and so forth. Furthermore, some patient risk factors (e.g., age, presence of kidney disease, any artery blockage, heart disease, etc.) can be included in the risk factors.

Prior to the specific treatment in question, an expert will have recorded an angioplasty with a patient suffering from kidney disease. During the recording of the mixed reality session, the case-based simulation recorder module **40** can annotate key events (automatically or by the expert) as a change to a standard procedure and patient-specifics/population data so that the risk model 34 can be attached. The mixed reality medical procedure educational simulation 32 is then generated and saved to the simulations database **30** , along with the anonymized associated patient data and machine data that tracked the different states of the IGT system and catheter.

The **MP** can then use the case selector module **42** to select the mixed reality medical procedure educational simulation **32** from the simulations database **30.**

When a **MP** wants to use the mixed reality medical procedure educational simulation **32**, the risk model **34** is modified/tailored to the physician's educational needs. The risk assessment module **44** performs this by considering the number of previous similar cases that have been conducted by the **MP** and filters out (reduced the probabilities), and compares this against statistically recorded data of previous similar cases/populations and their outcomes to quantify the risk. In the case of kidney disease, the amount of contrast agent used at key imaging moments (diagnosis of obstructed arteries/catheter placement/stent placement) can carry a high-risk probability with a number of possible outcomes during the procedure, each with their own probabilities. Also, a risk factor **36** in terms of the image quality is factored in when the contrast agent is too low.

Now that the risk model **34** and the **MP** experience is considered, the simulation creation module **46** can create the user-specific mixed reality medical procedure educational simulation **12** with the risk model **34** as input and will play out certain situations that will help educate the **MP** and increase their competence in dealing with such cases.

The **MP** places themselves into the simulation/mixed reality environment with the headset **14** (in this case, an IGT system) and proceeds to place the catheter whilst, adapt the contrast fluid and looking at the image during the medical procedure. During this, the **MP**'s pre-recorded view is overlaid to guide the **MP** when a particular risk-based event is triggered (too much contrast or poor-quality image). Moreover, the simulation feedforward playback module **48**, based on layout sensing, can hide additional details, such as and additional patient monitor, an additional technician with observing role so that the recorded scene fits optimally with the **MP** context to create a more realistic and matching experience.

Various Examples in accordance with the above disclosure are enumerated below. Example 1. A non-transitory computer readable medium (**26**) storing instructions executable by at least one electronic processor (**20**) to perform a mixed reality medical procedure simulation method (**100**), the method comprising:
receiving, via one or more user inputs from a user with at least one user input device (22), a medical procedure the user wants to simulate;
identifying, in a simulations database (**30**), a mixed reality medical procedure educational simulation (**32**) similar to the medical procedure the user wants to simulate;
performing a risk analysis on the medical procedure the user wants to simulate to generate a risk model (**34**) for the medical procedure the user wants to simulate;
generating a user-specific mixed reality medical procedure simulation (**12**) by augmenting the selected mixed reality medical procedure simulation with the risk model; and
presenting the user-specific mixed reality medical procedure simulation to the user.

Example 2. The non-transitory computer readable medium (**26**) of Example 1, wherein the risk analysis is based on one or more risk factors (**36**) of a specific patient on whom the user wants to perform the medical procedure the user want to learn.

Example 3. The non-transitory computer readable medium (**26**) of either one of Examples 1 and 2, wherein the risk analysis includes:
analyzing log data of a medical device to be used in the medical procedure the user wants to learn and historical patient data of historical patients who have undergone the medical procedure the user wants to learn; and
quantifying the one or more risk factors (**36**) based on the analyzing of the log data and the historical patient data.

Example 4. The non-transitory computer readable medium (**26**) of Example 3, wherein quantifying the one or more risk factors (**36**) based on the analyzing of the log data and the historical patient data further includes:
quantifying the one or more risk factors based on an expertise level of the user.

Example 5. The non-transitory computer readable medium (**26**) of either one of Examples 3 and 4, wherein the generating of the user-specific mixed reality medical procedure educational simulation (**12**) includes:
augmenting the selected mixed reality medical procedure educational simulation with the quantified one or more risk factors.

Example 6. The non-transitory computer readable medium (**26**) of Example 5, wherein augmenting the selected mixed reality medical procedure educational simulation (**32**) with the quantified one or more risk factors (**36**) includes:
adding mixed reality content indicative of the one or more risk factors at time points in the selected mixed reality medical procedure educational simulation at which the quantified one or more risk factors occur.

Example 7. The non-transitory computer readable medium (**26**) of any one of Examples 1-6, wherein presenting the user-specific mixed reality medical procedure educational simulation (**12**) to the user includes:
presenting the simulation using an AR device (**14**) worn by the user.

Example 8. The non-transitory computer readable medium (**26**) of any one of Examples 1-7, wherein presenting the user-specific mixed reality medical procedure educational simulation (**12**) to the user includes:
playing back the user-specific mixed reality medical procedure educational simulation;
during the playing back, receiving a user input indicative of the user interacting with the user-specific mixed reality medical procedure educational simulation; and
adjusting a speed of playback of the user-specific mixed reality medical procedure educational simulation based on the user input received during the playing back.

Example 9. The non-transitory computer readable medium (**26**) of Example 8, wherein presenting the user-specific mixed reality medical procedure educational simulation (**12**) to the user includes:
playing back the user-specific mixed reality medical procedure educational simulation;
during the playing back, receiving a user input indicative of the user interacting with the user-specific mixed reality medical procedure educational simulation;
determining whether the received user input corresponds to a correct step in the medical procedure; and
if the received user input is determined to not correspond to a correct step, providing mixed reality content that the user input was not correct.

Example 10. A non-transitory computer readable medium (**26**) storing instructions executable by at least one electronic processor (**20**) to perform an augmented reality (AR) medical procedure education method (**100**), the method comprising:
receiving, via one or more user inputs from a user with at least one user input device (**22**), a medical procedure the user wants to learn;
identifying, in a simulations database (**30**), a mixed reality medical procedure educational simulation (**32**) similar to the medical procedure the user wants to learn;
performing a risk analysis on the medical procedure the user wants to learn to generate a risk model (**34**) for the medical procedure the user wants to learn by quantifying one or more risk factors (**36**) based on an expertise level of the user;
generating a user-specific mixed reality medical procedure educational simulation (**12**) by augmenting the selected mixed reality medical procedure educational simulation with the risk model; and
presenting the user-specific mixed reality medical procedure educational simulation to the user.

Example 11. The non-transitory computer readable medium (**26**) of Example 10, wherein the risk analysis is based on one or more risk factors (**36**) of a specific patient on whom the user wants to perform the medical procedure the user want to learn.

Example 12. The non-transitory computer readable medium (**26**) of either one of Examples 10 and 11, wherein the risk analysis includes:
analyzing log data of a medical device to be used in the medical procedure the user wants to learn and historical patient data of historical patients who have undergone the medical procedure the user wants to learn; and
quantifying the one or more risk factors (**36**) based on the analyzing of the log data and the historical patient data.

Example 13. The non-transitory computer readable medium (**26**) of any one of Examples 10-12, wherein the generating of the user-specific mixed reality medical procedure educational simulation (**12**) includes:
augmenting the selected mixed reality medical procedure educational simulation with the quantified one or more risk factors.

Example 14. The non-transitory computer readable medium (**26**) of Example 13, wherein augmenting the selected mixed reality medical procedure educational simulation (**32**) with the quantified one or more risk factors (**36**) includes:
adding mixed reality content indicative of the one or more risk factors at time points in the selected mixed reality medical procedure educational simulation at which the quantified one or more risk factors occur.

Example 15. The non-transitory computer readable medium (**26**) of any one of Examples 10-14, wherein presenting the user-specific mixed reality medical procedure educational simulation (**12**) to the user includes:
presenting the simulation using an AR device (**14**) worn by the user.

Example 16. The non-transitory computer readable medium (**26**) of any one of Examples 10-15, wherein presenting the user-specific mixed reality medical procedure educational simulation (**12**) to the user includes:
playing back the user-specific mixed reality medical procedure educational simulation;
during the playing back, receiving a user input indicative of the user interacting with the user-specific mixed reality medical procedure educational simulation; and
adjusting a speed of playback of the user-specific mixed reality medical procedure educational simulation based on the user input received during the playing back.

Example 17. The non-transitory computer readable medium (**26**) of Example 16, wherein presenting the user-specific mixed reality medical procedure educational simulation (**12**) to the user includes:
playing back the user-specific mixed reality medical procedure educational simulation;
during the playing back, receiving a user input indicative of the user interacting with the user-specific mixed reality medical procedure educational simulation;
determining whether the received user input corresponds to a correct step in the medical procedure; and
if the received user input is determined to not correspond to a correct step, providing mixed reality content that the user input was not correct.

Example 18. A mixed reality medical procedure education method (**100**), the method comprising:
receiving, via one or more user inputs from a user with at least one user input device (**22**), a medical procedure the user wants to learn;
identifying, in a simulations database (**30**), a mixed reality medical procedure educational simulation (**32**) similar to the medical procedure the user wants to learn;
performing a risk analysis on the medical procedure the user wants to learn to generate a risk model (**34**) for the medical procedure the user wants to learn by analyzing log data of a medical device to be used in the medical procedure the user wants to learn and historical patient data of historical patients who have undergone the medical procedure the user wants to learn and quantifying the one or more risk factors (**36**) based on the analyzing of the log data and the historical patient data;
generating a user-specific mixed reality medical procedure educational simulation (**12**) by augmenting the selected mixed reality medical procedure educational simulation with the risk model; and
presenting the user-specific mixed reality medical procedure educational simulation to the user.

Example 19. The method (**100**) of Example 18, wherein the risk analysis is based on one or more risk factors (**36**) of a specific patient on whom the user wants to perform the medical procedure the user wants to learn.

Example 20. The method (**100**) of either one of Examples 18 and 19, wherein the generating of the user-specific mixed reality medical procedure educational simulation (**12**) includes:
augmenting the selected mixed reality medical procedure educational simulation with the quantified one or more risk factors.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A non-transitory computer readable medium (**26**) storing instructions executable by at least one electronic processor (**20**) to perform a mixed reality medical procedure simulation method (**100**), the method comprising:
receiving, via one or more user inputs from a user with at least one user input device (**22**), a medical procedure the user wants to simulate;
identifying, in a simulations database (**30**), a mixed reality medical procedure educational simulation (**32**) similar to the medical procedure the user wants to simulate;
performing a risk analysis on the medical procedure the user wants to simulate to generate a risk model (**34**) for the medical procedure the user wants to simulate;
generating a user-specific mixed reality medical procedure simulation (12) by augmenting the selected mixed reality medical procedure simulation with the risk model; and
presenting the user-specific mixed reality medical procedure simulation to the user.

2. The non-transitory computer readable medium (**26**) of claim 1, wherein the risk analysis is based on one or more risk factors (**36**) of a specific patient on whom the user wants to perform the medical procedure the user want to learn.

3. The non-transitory computer readable medium (**26**) of either one of claims 1 and 2, wherein the risk analysis includes:
analyzing log data of a medical device to be used in the medical procedure the user wants to learn and historical patient data of historical patients who have undergone the medical procedure the user wants to learn; and
quantifying the one or more risk factors (**36**) based on the analyzing of the log data and the historical patient data.

4. The non-transitory computer readable medium (**26**) of claim 3, wherein quantifying the one or more risk factors (**36**) based on the analyzing of the log data and the historical patient data further includes:
quantifying the one or more risk factors based on an expertise level of the user.

5. The non-transitory computer readable medium (**26**) of either one of claims 3 and 4, wherein the generating of the user-specific mixed reality medical procedure educational simulation (**12**) includes:
augmenting the selected mixed reality medical procedure educational simulation with the quantified one or more risk factors.

6. The non-transitory computer readable medium (**26**) of claim 5, wherein augmenting the selected mixed reality medical procedure educational simulation (**32**) with the quantified one or more risk factors (**36**) includes:
adding mixed reality content indicative of the one or more risk factors at time points in the selected mixed reality medical procedure educational simulation at which the quantified one or more risk factors occur.

7. The non-transitory computer readable medium (**26**) of any one of claims 1-6, wherein presenting the user-specific mixed reality medical procedure educational simulation (**12**) to the user includes:
presenting the simulation using an AR device (**14**) worn by the user.

8. The non-transitory computer readable medium (**26**) of any one of claims 1-7, wherein presenting the user-specific mixed reality medical procedure educational simulation (**12**) to the user includes:
playing back the user-specific mixed reality medical procedure educational simulation;
during the playing back, receiving a user input indicative of the user interacting with the user-specific mixed reality medical procedure educational simulation; and
adjusting a speed of playback of the user-specific mixed reality medical procedure educational simulation based on the user input received during the playing back.

9. The non-transitory computer readable medium (**26**) of claim 8, wherein presenting the user-specific mixed reality medical procedure educational simulation (**12**) to the user includes:
playing back the user-specific mixed reality medical procedure educational simulation;
during the playing back, receiving a user input indicative of the user interacting with the user-specific mixed reality medical procedure educational simulation;
determining whether the received user input corresponds to a correct step in the medical procedure; and
if the received user input is determined to not correspond to a correct step, providing mixed reality content that the user input was not correct.
